# EUROPEAN PATENT APPLICATION

(11) **EP 1 712 891 A2**
(43) Date of publication of application: **18.10.2006**
(21) Application number: 05020589.7
(22) Date of filing: 21.09.2005
(51) Int. Cl.: G01N 13/12, G01N 27/447, G12B 21/04

(54) **Molecular resonant tunneling sensor and methods of fabricating and using the same**

(30) Priority: 15.04.2005 US 107461
(71) Applicant: Agilent Technologies, Inc., A Delaware Corporation, Palo Alto, CA 94306 (US)
(72) Inventor: Barth, Phillip W., Loveland Colorado 80537-0599 (US); Myerholtz, Carl A., Loveland Colorado 80537-0599 (US)
(74) Representative: Schoppe, Fritz

(57) **Abstract**

Resonant tunneling devices and methods of using and fabricating the same are provided. The subject devices include a first **110** and second **111** fluid containment members separated by a fluid barrier **109** having a single nanopore **101** therein providing fluid communication between the first and second fluid containment members, wherein the nanopore **101** has a top inner diameter that is smaller than a bottom inner diameter and includes first **107** and second **105** perimeter electrodes separated by an insulator element **106,** and a proteinaceous channel **102** positioned in the nanopore. Also provided are methods of fabricating such a device and methods of using such a device for improved detection and characterization of a sample.

## Description

### BACKGROUND OF THE INVENTION

Techniques for manipulating matter at the nanometer scale ("nanoscale") are important for many electronic, chemical and biological purposes (See Li et al., "Ion beam sculpting at nanometer length scales", Nature, 412: 166-169, 2001). Among such purposes are the desire to more quickly sequence biopolymers such as DNA. Nanopores, both naturally occurring and artificially fabricated, have recently attracted the interest of molecular biologists and biochemists for the purpose of DNA sequencing.

It has been demonstrated that a voltage gradient can drive a biopolymer such as single-stranded DNA (ssDNA) in an aqueous ionic solution through a naturally occurring trans-substrate channel, or "nanopore," such as a α-hemolysin pore in a lipid bilayer. (See Kasianowicz et al., "Characterization of individual polynucleotide molecules using a membrane channel", Proc. Natl. Acad. Sci. USA, 93: 13770-13773, 1996). The process in which the DNA molecule goes through the pore has been dubbed "translocation". During the translocation process, the extended biopolymer molecule blocks a substantial portion of the otherwise open nanopore channel. This blockage decreases the ionic electrical current flow occurring through the nanopore in the ionic solution. The passage of a single biopolymer molecule can, therefore, be monitored by recording the translocation duration and the decrease in current. Many such events occurring sequentially through a single nanopore provide data that can be plotted to yield useful information concerning the structure of the biopolymer molecule. For example, given uniformly controlled translocation conditions, the length of the individual biopolymer can be estimated from the translocation time.

One desire of scientists is that the individual monomers of the biopolymer strand might be identified via the characteristics of the blockage current, but this hope may be unrealized because of first-principle signal-to-noise limitations and because the naturally occurring nanopore is thick enough that several monomers of the biopolymer are present in the nanopore simultaneously.

More recent research has focused on fabricating artificial nanopores. Ion beam sculpting using a diffuse beam of low-energy argon ions has been used to fabricate nanopores in thin insulating substrates of materials such as silicon nitride (See Li et al., "Ion beam sculpting at nanometer length scales", Nature, 412: 166-169, 2001). Double-stranded DNA (dsDNA) has been passed through these artificial nanopores in a manner similar to that used to pass ssDNA through naturally occurring nanopores. Current blockage data obtained with dsDNA is reminiscent of ionic current blockages observed when ssDNA is translocated through the channel formed by α-hemolysin in a lipid bilayer. The duration of these blockages has been on the millisecond scale and current reductions have been to 88% of the open-pore value. This observation is commensurate with translocation of a rod-like molecule whose cross-sectional area is 3-4 nm² (See Li et al., "Ion beam sculpting at nanometer length scales", Nature, 412: 166-169, 2001). However, as is the case with single-stranded biopolymers passing through naturally occurring nanopores, first-principle signal-to-noise considerations make it difficult or impossible to obtain information on the individual monomers in the biopolymer.

Because of the potential applicability of nanopore devices for a variety of different applications, there is continued interest in the development of new nanopore device structures and methods of using the same.

### SUMMARY OF THE INVENTION

Resonant tunneling devices and methods of using and fabricating the same are provided. The subject devices include a first and second fluid containment members separated by a fluid barrier having a single nanopore therein providing fluid communication between the first and second fluid containment members. The single nanopore has a top inner diameter that is smaller than a bottom inner diameter and includes first and second perimeter electrodes separated by an insulator element, and a proteinaceous channel positioned in the nanopore. Also provided are methods of fabricating such a device and methods of using such a device for improved detection and characterization of a sample.

A feature of the present invention provides a device including a first and second fluid containment members separated by a fluid barrier having a single nanopore therein providing fluid communication between the first and second fluid containment members. The nanopore has a top inner diameter that is smaller than a bottom inner diameter and includes first and second perimeter electrodes separated by an insulator element. Also present is a proteinaceous channel positioned in the nanopore. In some embodiments, the nanopore has inner walls configured to define a frustum. In certain embodiments the ratio of the length of the top inner diameter to the length of the bottom inner diameter ranges from about 0.05 to about 1.0. In further embodiments, the top inner diameter has a length ranging from about 15 to about 40 nm. In yet further embodiments, the bottom inner diameter has a length ranging from about 20 to about 100 nm.

In some embodiments, the first and second perimeter electrodes are part of a resonant tunneling sensor. In further embodiments the first and second perimeter electrodes are within a distance of about 2 to about 8 nm from the top inner diameter. In some embodiments the device further includes an element for applying an electrical field between the first and second perimeter electrodes. In additional embodiments, the device further includes an element for measuring an electrical field between the first and second perimeter electrodes.

In some embodiments, the fluid barrier comprises silicon nitride, the first and second perimeter electrodes comprise platinum and the insulator element comprises silicon dioxide. In further embodiments, the proteinaceous channel is synthetic channel or a naturally occurring channel, such as a heptameric channel of α-hemolysin. In some embodiments, the proteinaceous channel is held in position with a lipid bilayer.

Another feature of the invention provides a method for fabricating a nanopore in a solid substrate, including producing a nanodimensioned passageway through a planar solid substrate, positioning an electrode element about an opening of the passageway, wherein the electrode element includes first and second perimeter electrodes separated by an insulator element, and positioning a proteinaceous channel in the nanodimensioned passageway to produce the nanopore. In some embodiments, the electrode element is positioned about the opening such that the ring electrodes are coaxial with the opening. In further embodiments the nanodimensioned passageway is produced in the planar solid substrate using a focused ion beam protocol.

In some embodiments, the electrode element is positioned about the passageway by sequentially depositing about the opening a first conductive element, an insulator element, and a second conductive element. In some embodiments, the deposited element overhangs a preceding element such that the nanopore has a top inner diameter that is smaller than a bottom inner diameter. In further embodiments, the sequentially depositing step includes using a molecular beam epitaxy protocol. In certain embodiments, the nanopore has inner walls that define a frustum. In some embodiments, the electrode element is a resonant tunneling sensor. In some embodiments, the proteinaceous channel is positioned in the nanodimensioned passageway by using a lipid bilayer.

Yet another feature of the invention provides a method including applying an electrical current between first and second perimeter electrodes of a device including a first and second fluid containment members separated by a fluid barrier having a single nanopore therein providing fluid communication between the first and second fluid containment members, wherein the nanopore has a top inner diameter that is smaller than a bottom inner diameter and includes first and second perimeter electrodes separated by an insulator element, a proteinaceous channel positioned in the nanopore, and monitoring the electrical current though the nanopore.

In some embodiments, the monitoring is performed over a period of time. In some embodiments, the monitoring is performed in the presence of a polymeric compound in the first fluid containment chamber of the device. In further embodiments, the polymeric compound is a nucleic acid. In some embodiments, the method is a method of characterizing a polymeric compound. In further embodiments, the method of characterizing is a method of sequencing a nucleic acid.

### BRIEF DESCRIPTION OF THE DRAWINGS

The invention is best understood from the following detailed description when read in conjunction with the accompanying drawings. It is emphasized that, according to common practice, the various features of the drawings are not to-scale. On the contrary, the dimensions of the various features are arbitrarily expanded or reduced for clarity. Included in the drawings are the following figures:
FIG. 1 is a schematic representation of a cross-section of an embodiment 100 of the present invention.
FIG. 2 is a schematic representation of a cross-section of an embodiment 100 of the present invention with additional elements.
FIGS. 3A to 3J illustrate the sequential steps of a method of fabricating embodiment 100 of the present invention.
FIGS. 4A to 4D illustrate a method of angled line-of-sight layer deposition used in fabricating embodiment 100 of the present invention.

### DEFINITIONS

A "biopolymer" is a polymer of one or more types of repeating units, regardless of the source (e.g., biological (e.g., naturally-occurring, obtained from a cell-based recombinant expression system, and the like) or synthetic). Biopolymers may be found in biological systems and particularly include polypeptides, polynucleotides, proteoglycans; etc., including compounds containing amino acids, nucleotides, or a mixture thereof.

The terms "polypeptide" and "protein" are used interchangeably throughout the application and mean at least two covalently attached amino acids, which includes proteins, polypeptides, oligopeptides and peptides. A polypeptide may be made up of naturally occurring amino acids and peptide bonds, synthetic peptidomimetic structures, or a mixture thereof. Thus "amino acid", or "peptide residue", as used herein encompasses both naturally occurring and synthetic amino acids. For example, homo-phenylalanine, citrulline and noreleucine are considered amino acids for the purposes of the invention. "Amino acid" also includes imino acid residues such as proline and hydroxyproline. The side chains may be in either the D- or the L-configuration.

In general, biopolymers, e.g., polypeptides or polynucleotides, may be of any length, e.g., greater than 2 monomers, greater than 4 monomers, greater than about 10 monomers, greater than about 20 monomers, greater than about 50 monomers, greater than about 100 monomers, greater than about 300 monomers, usually up to about 500, 1000 or 10,000 or more monomers in length. "Peptides" and "oligonucleotides" are generally greater than 2 monomers, greater than 4 monomers, greater than about 10 monomers, greater than about 20 monomers, usually up to about 10, 20, 30, 40, 50 or 100 monomers in length. In certain embodiments, peptides and oligonucleotides are between 5 and 30 amino acids in length.

The terms "polypeptide" and "protein" are used interchangeably herein. The term "polypeptide" includes polypeptides in which the conventional backbone has been replaced with non-naturally occurring or synthetic backbones, and peptides in which one or more of the conventional amino acids have been replaced with one or more non-naturally occurring or synthetic amino acids. The term "fusion protein" or grammatical equivalents thereof references a protein composed of a plurality.of polypeptide components, that while typically not attached in their native state, typically are joined by their respective amino and carboxyl termini through a peptide linkage to form a single continuous polypeptide. Fusion proteins may be a combination of two, three or even four or more different proteins. The term polypeptide includes fusion proteins, including, but not limited to, fusion proteins with a heterologous amino acid sequence, fusions with heterologous and homologous leader sequences, with or without N-terminal methionine residues; immunologically tagged proteins; fusion proteins with detectable fusion partners, e.g., fusion proteins including as a fusion partner a fluorescent protein, β-galactosidase, luciferase, and the like.

A "monomeric residue" of a biopolymer is a subunit, i.e., monomeric unit, of a biopolymer. Nucleotides are monomeric residues of polynucleotides and amino acids are monomeric residues of polypeptides.

A "substrate" refers to any surface that may or may not be solid and which is capable of holding, embedding, attaching or which may comprise the whole or portions of an excitable molecule.

The term "nanopore" refers to a pore or hole having a minimum diameter on the order of nanometers and extending through a thin substrate. Nanopores can vary in size and can range from 1 nm to around 300 nm in diameter. In representative embodiments, nanopores have been roughly around 1.5 nm to 30 nm, e.g., 3 nm -20 nm in diameter. The thickness of the substrate through which the nanopore extends can range from 1 nm to around 700 nm.

A biopolymer that is "in", "within" or moving through a nanopore means that the entire biopolymer or any portion thereof, may located within the nanopore.

The term "resonant tunneling" refers to refers to the quantum mechanical tunneling of electrons from one electrode to another electrode through quantum well states formed between the two electrodes, and may be detected as enhanced conduction as seen in a plot of the differential of current with respect to voltage when plotted versus applied voltage, i.e., a peak in either I versus V or in dI/dV versus V, where I is current, V is applied voltage, and dI/dV is the differential of current with respect to voltage.

The term "ramping potential" or "bias potential" refers to having the ability to establish a variety of different voltages over time. In certain cases, this may be referred to as "scanning a voltage" or providing a voltage which varies over time. A ramping potential may provided by a "ramping potential-providing element" or a "potential-providing element".

The term "voltage gradient" refers to a gradient of potentials between any two electrodes.

The term "tunneling" refers to the ability of an electron to move from a first position in space to a second position in space through a region that would be energetically excluded without quantum mechanical tunneling.

"Hybridizing", "annealing" and "binding", with respect to polynucleotides, are used interchangeably. "Binding efficiency" refers to the productivity of a binding reaction, measured as either the absolute or relative yield of binding product formed under a given set of conditions in a given amount of time. "Hybridization efficiency" is a particular sub-class of binding efficiency, and refers to binding efficiency in the case where the binding components are polynucleotides.

It will also be appreciated that throughout the present application, that words such as "first", "second" are used in a relative sense only. A "set" may have one type of member or multiple different types. "Fluid" is used herein to reference a liquid.

The terms "symmeCric" and "symmetrized' refer to the situation in which the tunneling barriers from each electrode to the biopolymer are substantially equal in magnitude.

The terms "translocation" and "translocate" refer to movement through a nanopore from one side of the substrate to the other, the movement occurring in a defined direction.

The terms "portion" and "portion of a biopolymer" refer to a part, subunit, monomeric unit, portion of a monomeric unit, atom, portion of an atom, cluster of atoms, charge or charged unit.

In certain embodiments, the methods are coded onto a computer-readable medium in the form of "programming", where the term "computer readable medium" as used herein refers to any storage or transmission medium that participates in providing instructions and/or data to a computer for execution and/or processing. Examples of storage media include floppy disks, magnetic tape, CD-ROM, a hard disk drive, a ROM or integrated circuit, a magneto-optical disk, or a computer readable card such as a PCMCIA card and the like, whether or not such devices are internal or external to the computer. A file containing information may be "stored" on computer readable medium, where "storing" means recording information such that it is accessible and retrievable at a later date by a computer.

With respect to computer readable media, "permanent memory" refers to memory that is permanent. Permanent memory is not erased by termination of the electrical supply to a computer or processor. Computer hard-drive ROM (i.e. ROM not used as virtual memory), CD-ROM, floppy disk and DVD are all examples of permanent memory. Random Access Memory (RAM) is an example of non-permanent memory. A file in permanent memory may be editable and re-writable.

A "computer-based system" refers to the hardware means, software means, and data storage means used to analyze the information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means may comprise any manufacture comprising a recording of the present information as described above, or a memory access means that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" references any hardware and/or software combination that will perform the functions required of it. For example, any processor herein may be a programmable digital microprocessor such as available in the form of an electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

"Communicating" information means transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data. The data may be transmitted to the remote location for further evaluation and/or use. Any convenient telecommunications means may be employed for transmitting the data, e.g., facsimile, modem, internet, etc.

The term "adjacent" refers to anything that is near, next to or adjoining. For instance, a nanopore referred to as "adjacent to an excitable molecule" may be near an excitable molecule, it may be next to the excitable molecule, it may pass through an excitable molecule or it may be adjoining the excitable molecule. "Adjacent" can refer to spacing in linear, two-dimensional and three-dimensional space. In general, if a quenchable excitable molecule is adjacent to a nanopore, it is sufficiently close to the edge of the opening of the nanopore to be quenched by a biopolymer passing through the nanopore. Similarly, electrodes that are positions adjacent to a nanopore are positioned such that resonance tunneling occurs a biopolymer passes through the nanopore. Compositions that are adjacent may or may not be in direct contact.

If one composition is "bound" to another composition, the bond between the compositions does not have to be in direct contact with each other. In other words, bonding may be direct or indirect, and, as such, if two compositions (e.g., a substrate and a nanostructure layer) are bound to each other, there may be at least one other composition (e.g., another layer) between the compositions. Binding between any two compositions described herein may be covalent or non-covalent.

The term "assessing" includes any form of measurement, and includes determining if an element is present or not. The terms "determining", "measuring", "evaluating", "assessing" and "assaying" are used interchangeably and may include quantitative and/or qualitative determinations. Assessing may be relative or absolute. "Assessing the presence of" includes determining the amount of something present, and/or determining whether it is present or absent.

### DETAILED DESCRIPTION OF THE INVENTION

Resonant tunneling devices and methods of using and fabricating the same are provided. The subject devices include a first and second fluid containment members separated by a fluid barrier having a single nanopore therein providing fluid communication between the first and second fluid containment members. The nanopore has a top inner diameter that is smaller than a bottom inner diameter and includes first and second perimeter electrodes separated by an insulator element, and a proteinaceous channel positioned in the nanopore. Also provided are methods of fabricating such a device and methods of using such a device for improved detection and characterization of a sample.

Before the present invention is described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "an", and "the" include plural referents unless the context clearly dictates otherwise. Thus, for example, reference to "a biopolymer" includes a plurality of such biopolymers and reference to "the electrode" includes reference to one or more electrodes and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention.

### The Subject Devices

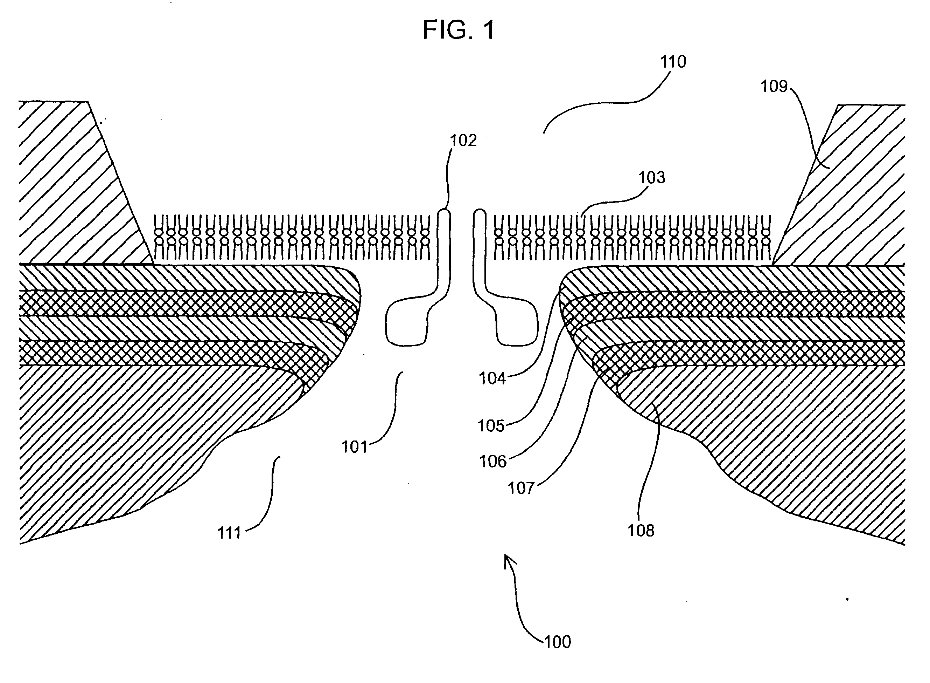

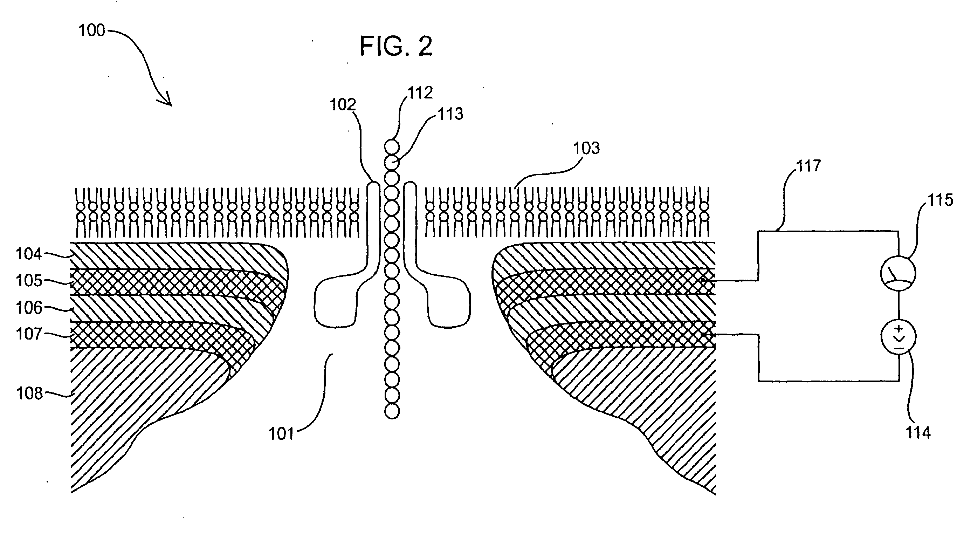

The present invention provides devices including a resonant tunneling sensor and a proteinaceous channel positioned in the resonant tunneling sensor. FIGS. 1 and 2 illustrate cross-sections of an embodiment 100 of the present invention and are used in the flowing description. In general, the device of the present invention includes a first 110 and second 111 fluid containment members separated by a fluid barrier 109 having a single nanopore therein 101 providing fluid communication between the first 110 and second 111 fluid containment members, wherein the nanopore has a top inner diameter that is smaller than the inner diameter and includes a first 107 and 105 second perimeter electrodes separated by an insulating element 106, and a proteinaceous channel 102 positioned in the nanopore..

The fluid barrier, or substrate 108, may comprise of a variety of materials known in the art for designing substrates and nanopores. A substrate suitable for use with the subject device may include one or more layers of one or more materials including, but not limited to, silicon nitride, silicon dioxide, platinum or other metals, silicon oxynitride, silicon rich nitride, organic polymers, and other insulating layers, carbon based materials, plastics, metals, or other materials known in the art for etching or fabricating semiconductor or electrically conducting materials. A suitable substrate need not be of uniform thickness. The substrate may or may not be a solid material, and for example may comprise in part or in whole a mesh, wire, or other material in which a nanodimensional passageway, such as nanopore, may be constructed. The substrate may comprise various shapes and sizes. However, it must be large enough and of sufficient width to be capable of forming the nanopore through it. In representative embodiments, the substrate has a width ranging from about 3 mm to about 30 mm, such as from about 4 mm to about 20 mm, including about 6 mm to about 12 mm. In addition, the substrate may comprise of various structural properties, such as rigid or flexible. However, the substrate must be sufficiently rigid enough to support the elements of the device and capable of forming the nanopore through it.

The nanopore 101 may be positioned anywhere on or through the fluid barrier, such as the substrate. The nanopore may have a diameter ranging in size from about 1 nm to as large as 300 nm. In representative embodiments, the nanopore is sufficiently large enough to allow a proteinaceous channel to be positioned therein. In some embodiments, the nanopore has a diameter that ranges from about 10 nm to about 200 nm, including from about 20 nm to about 190 nm, such as from about 30 nm to about 175 nm.

Nanopores suitable for use with the subject devices have, in representative embodiments, a top inner diameter that is smaller than a bottom inner diameter. In certain embodiments, the inner walls of the nanopores will be configured to define a frustum structure, such as a frustoconical configuration. In certain embodiments the ratio of the length of the top inner diameter to the length of the bottom inner diameter will range form about 0.05 to about 1.0, including about 0.06 to about 0.9, about 0.1 to about 0.8, about 0.2 to about 0.7, such as about 0.3 to about 0.6, about 0.4 to about 0.5. In some embodiments, the nanopores of the present device will have a top inner diameter that has a length ranging from about 15 to about 40 nm, including about 18 nm to about 35 nm, about 20 nm to about 32 nm, 22 nm to about 30 nm, such as about 24 nm to about 28 nm. In some embodiments the nanopores of the present device will have a bottom inner diameter that has a length ranging from about 20 to about 100 nm, including about 25 nm to about 90 nm, about 30 nm to about 80 nm, 40 nm to about 70 m, such as bout 45 nm to about 65 nm, about 50 nm to about 60 nm.

Nanopores suitable for use with the subject device will also include first 107 and second 105 perimeter electrodes separated by an insulating element 106. As used herein "perimeter" is meant to include the continuous circumference of the nanopore. In other words, no portion of the perimeter will be left exposed. Accordingly, a suitable nanopore will include a first electrode 107 and second electrode 105 that surround the perimeter of the nanopore opening and are separated by an insulating element 106. In some embodiments, the first 107 and second 105 perimeter electrodes and the insulating element 106 will be stacked upon one another. In other words, the three layers will be stacked upon each other to form a sandwiched configuration, wherein a first electrode element 107 is provided, an insulating element 106 is provided on top of the first electrode element 107, and a second electrode element 105 is then provided over the insulating element. In certain embodiments, a second insulating element 104 is present on top of the second electrode element 105. This should not be interpreted to mean that the embodiment shown in FIG. 1 in any way will limit the spatial orientation and positioning of each of the components of the invention. However, the design must be capable of establishing a potential between the first electrode element 107, and the second electrode element 105 of the nanopore 101.

In some embodiments, the first 107 and second 105 perimeter electrodes and the insulating material 106 will have a thickness ranging from about 1 nm to about 10 nm, including from about 2 nm to about 9 nm, from about 3 nm to about 8 nm, from about 4 nm to about 7 nm, such as from about 5 nm to about 6 nm. In certain embodiments, the first 107 and second 105 perimeter electrodes are within a distance of from about 2 nm to about 15 nm from the top inner diameter of the nanopore, including from about 3 nm to about 12 nm, about 4 nm to about 11 nm, about 5 nm to about 10 nm, such as about 6 nm to about 9 nm, about 7 nm to about 8 nm.

In certain embodiments, the 107 first and 105 second perimeter electrodes of the nanopore 101 are part of a resonant tunneling sensor. The phrase "resonant tunneling" refers to the quantum tunneling of electrons from one electrode to another electrode through quantum well states formed between the two electrodes, and may be detected as enhanced conduction as seen in a plot of the differential of current with respect to voltage when plotted versus applied voltage, i.e., a peak in I versus V or in dI/dV versus V, where I is current, V is applied voltage, and dI/dV is the differential of current with respect to voltage.

The first 107 and second 105 perimeter electrodes may be made up of a variety of electrically conductive materials. Such materials include, but are not limited to, metals, silicides, organic conductors and superconductors, electrically conductive metals and alloys of tin, copper, zinc, iron, magnesium, cobalt, nickel, platinum and vanadium. Other materials well known in the art that provide for electrical conduction may also be employed. The insulating element 106 may be made up of a variety of materials that provide for insulation between the first 107 and second 105 perimeter electrode elements. A variety of suitable materials are well known in the art and may be used with the subject device. Representative materials include, for example, silicon dioxide, silicon nitride, silicon oxynitride, silicon rich nitride, organic polymers, and plastics, etc.

As noted above, the subject device 100 of the present invention also includes a proteinaceous channel 102, positioned in the nanopore 101. Proteinaceous channels suitable for use with the subject invention include naturally occurring and synthetic proteinaceous channels, including such channels that are well known in the art. By "proteinaceous" is meant that the channel 102 is made up of one or more, usually a plurality, of different proteins associated with each other to produce a channel having an inner diameter of appropriate dimensions. Suitable channels include porins, gramicidins, and synthetic peptides. Of particular interest is the heptameric nanopore or channel produced from α-hemolysin, particularly α-hemolysin from Staphylococcus aureus, where the channel is preferably rectified, by which is meant that the amplitude of the current flowing in one direction through the channel exceeds the amplitude of the current flowing through the channel in the opposite direction.

In certain embodiments, the proteinaceous channel of the device 100 is positioned in the nanopore 101 by using a lipid bilayer 103. A variety of different lipid bilayers are known in the art and may be used to position the proteinaceous channel 102 in the nanopore 101 of the device 100. Representative lipid bilayers include those prepared from one or more lipids of the following group, phosphatidlycholine, phosphatidylserine, phosphatidylethanolamine, glycerol monooleate, cholesterol, etc.

Referring to FIG. 2, the subject device 100, in some embodiments, will further include an element 114 for applying an electrical voltage between the first 107 and second 105 perimeter electrodes. The electrical voltage generating element 114 may be positioned anywhere relative to the substrate 108, the nanopore 101, the first perimeter electrode 107 and the second perimeter electrode 105. The electrical voltage generating element 114 should be capable of ramping to establish a time varying voltage between the first perimeter electrode 107 and the second perimeter electrode 105. A variety of electrical voltage generating element 114 may be employed with the present invention. A number of these electrical voltage generating elements 114 are known in the art. The electrical voltage generating element 114 has the ability to ramp to establish a time varying voltage between the first perimeter electrode 107 and the second perimeter electrode 105.

In certain embodiments, the subject device 100, will further include an element 115 for measuring an electrical current between the first 107 and second 105 perimeter electrodes. The electrical current measuring element 115, may be any structure, component or apparatus that is well known in the art and that may be electrically connected 117 to one or more components of the present invention. The device may further include other elements of the output generating system, including data acquisition software, an electronic storage medium, etc.

### Fabrication of the Subject Devices

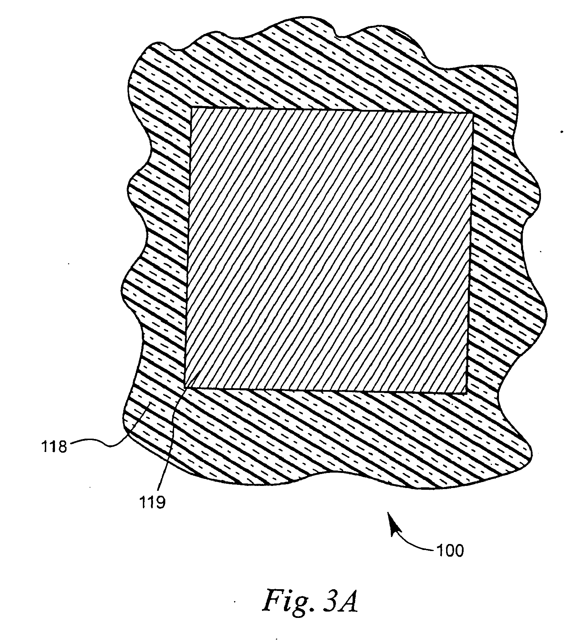

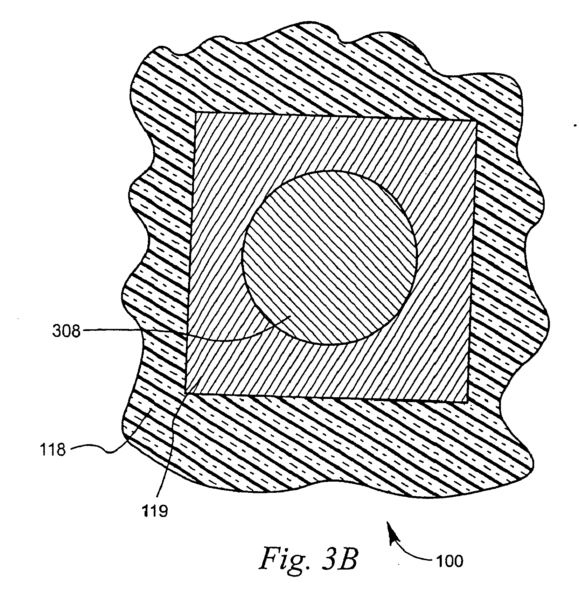

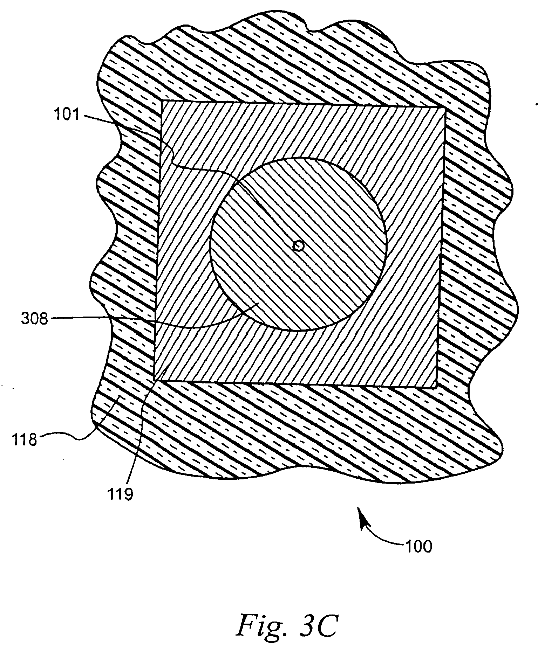

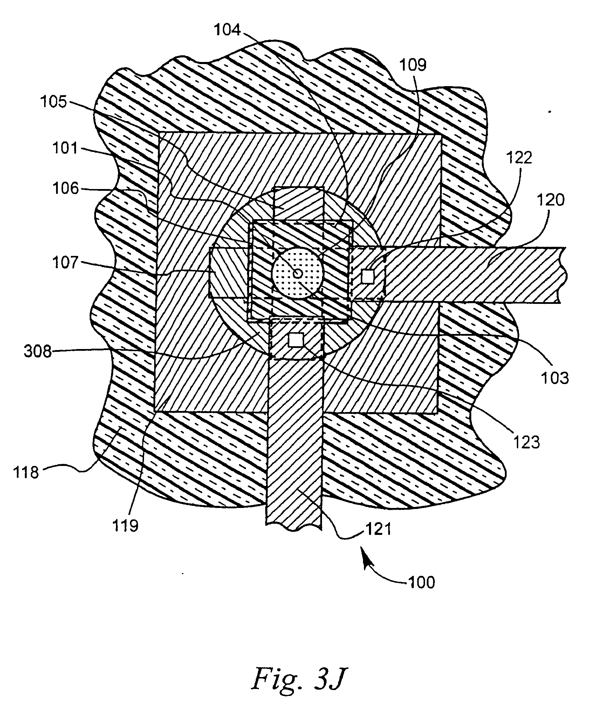

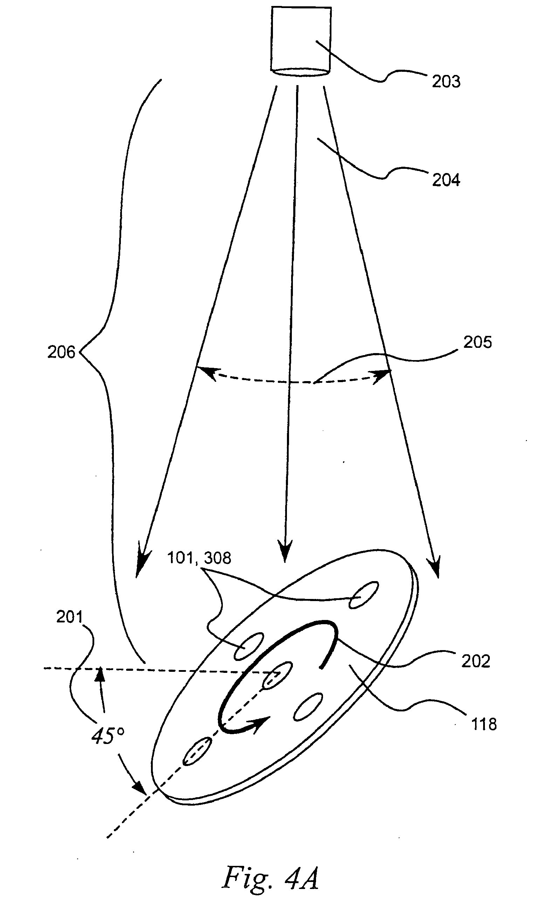

Having described representative embodiments of the device of the invention, a description of representative embodiments of methods of fabrication of the invention is now provided. A non-limiting exemplary method of fabricating an embodiment of the subject device 100 is provided in FIGS. 3A to 3J and 4A to 4D. The figures are not necessarily drawn to scale. For example, the diameter of the nanopore is exaggerated in order to make it visible at the drawing scale. In general fabrication of the subject device 100 includes producing a nanodimensioned passageway through a planar solid substrate, then positioning an electrode element about an opening of the passageway, wherein the electrode element includes first and second perimeter electrodes separated by an insulator element; and then positioning a proteinaceous channel in the nanodimensioned passageway to produce the nanopore.

In an exemplary embodiment, fabrication begins by forming in a substrate 118 a composite window 119 comprising a layer of silicon nitride typically 200 nm thick on top of a layer of silicon dioxide typically 500 nm thick, both layers forming a cladding layer on the exterior surfaces of a silicon wafer. The fabrication of this window 119 may be accomplished by the well-known steps of photolithography and etching of a hole in a silicon nitride layer on the bottom side of a substrate 118 such as a wafer of silicon, followed by etching of the substrate 118 in a hot aqueous caustic solution such as tetramethyl ammonium hydroxide (TMAH) in water. The caustic etching process removes the silicon beneath the window 119 but leaves the silicon dioxide and silicon nitride layers, resulting in the layout structure illustrated in FIG. 3A. Window 119 as drawn is 40 micrometers (µm) on a side, but may be larger or smaller.

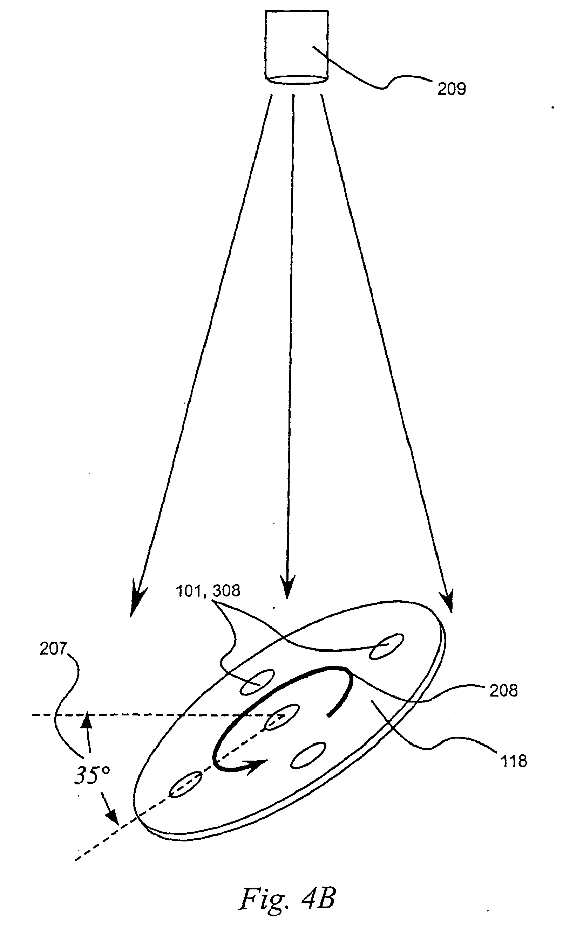

Next a photolithography step is performed to open a window in photoresist, and the silicon nitride layer is etched away to leave silicon dioxide window 308 as illustrated in FIG. 4B. This etching step is performed using well-known plasma etching techniques employing carbon tetrafluoride (CF₄), oxygen, and nitrogen to achieve a much faster etch rate for silicon nitride than for silicon dioxide. The technique of fabricating a silicon dioxide window region within a larger window region of silicon nitride on silicon dioxide is a separate invention, useful for obtaining a small, well-supported region of silicon dioxide with advantageous wetting properties as compared to silicon nitride.

Next, drilling is performed in the center of window 308 using a focused ion beam (FIB) of gallium ions, resulting in a nanodimensioned passageway with a diameter on the order of 50-200 nm extending through the thickness of the silicon dioxide layer. This FIB drilling process is followed by a published process of ion beam sculpting using a low energy beam of argon ions which acts to reduce the diameter of the edge of the nanoscale hole near proximate to the ion beam. This process is monitored by monitoring current of argon ions through the nanoscale hole, and is terminated when it has resulted in a nanodimensioned passageway 101 with a diameter ranging from about 10 nm to about 100 nm, as illustrated in FIG. 3C.

Next, a process of angled line-of-sight deposition as illustrated in FIG. 4A is used to deposit a layer of material used to form first perimeter electrode 107. FIG. 4A depicts an example of substrate 118 supporting multiple instances of oxide window 308 surrounding multiple instances of nanodimensioned passageways 101. Substrate 118 is tilted at an angle 201 so that its surface is, for example, 45 degrees from horizontal, and is rotated in a direction 202. Deposition source 203 is typically a vacuum evaporation source or a molecular beam epitaxy source or a sputtering source, and deposition stream 204 has some angular dispersion 205 as it travels along an average deposition path length 206. The result of the tilt and the rotation is that the deposited layer resulting from deposition stream 204 overhangs the edge of nanodimensioned passageway 101 The thickness of the layer deposited as shown in FIG. 4A ranges from about 1 nm to about 10 nm, such as about 2 nm, and the deposited material is in some embodiments platinum.

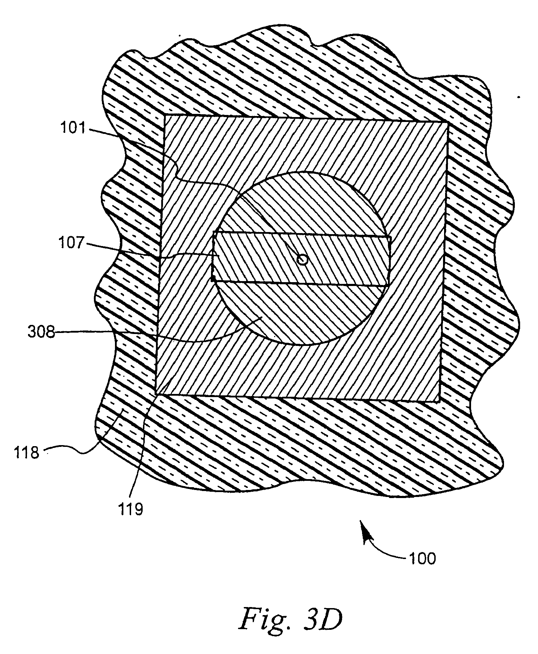

Next, a lithography step is performed, and etching is performed in a dilute solution of aqua regia, comprising a mixture of hydrochloric acid and nitric acid, to define the lateral extent of the first perimeter electrode 107 as illustrated in FIG. 3D. Alternatively, a photolithography step may be performed prior to the deposition step as illustrated in FIG. 4A and first perimeter electrode 107 may be defined by means of a lift-off process. In some embodiments, the first peripheral electrode element 107 may range in width from about 2 µm to about 7 µm, such as about 4 µm to about 6 µm, including about 5µm.

Next, a second angled line-of-sight deposition is performed as illustrated in FIG. 4B to form an insulating layer that will comprise insulator element 106. Angle 207 is less than angle 201, for example 35 degrees, and source 209 is typically a molecular beam epitaxy source or a sputtering source. The result of the deposition step of FIG. 4B is an insulator layer, in some embodiments comprising silicon dioxide. The insulating element 106 may range in thickness form about 1 nm to about 10 nm, including about 2 nm to about 9 nm, about 3 nm to about 8 nm, 4 nm to about 7 nm, such as about 5 nm to about 6 nm. In certain embodiments, the insulating element 106 overhangs the edge of nanopore 101 but which, as illustrated in FIGS. 1 and 2, does not occlude the overhanging perimeter the first perimeter electrode 107 because the perimeter of electrode element 107 is shadowed from the deposition stream during the line-of-sight deposition.

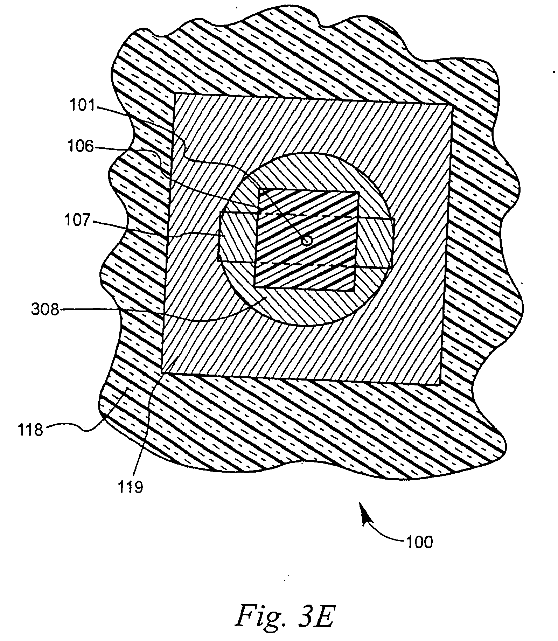

Next, a lithography step is performed and etching is performed in a dilute solution of buffered hydrofluouric acid called "buffered oxide etch" or "B.O.E," to define the lateral extent of optional insulator element 106 as shown in FIG. 3E. Alternatively, a photolithography step may be performed prior to the deposition step as illustrated in FIG. 4B and the optional insulator element 106 may be defined by means of a lift-off process.

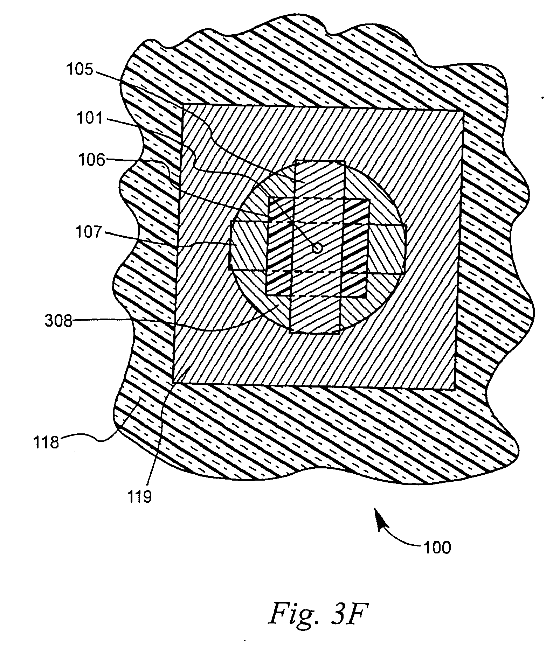

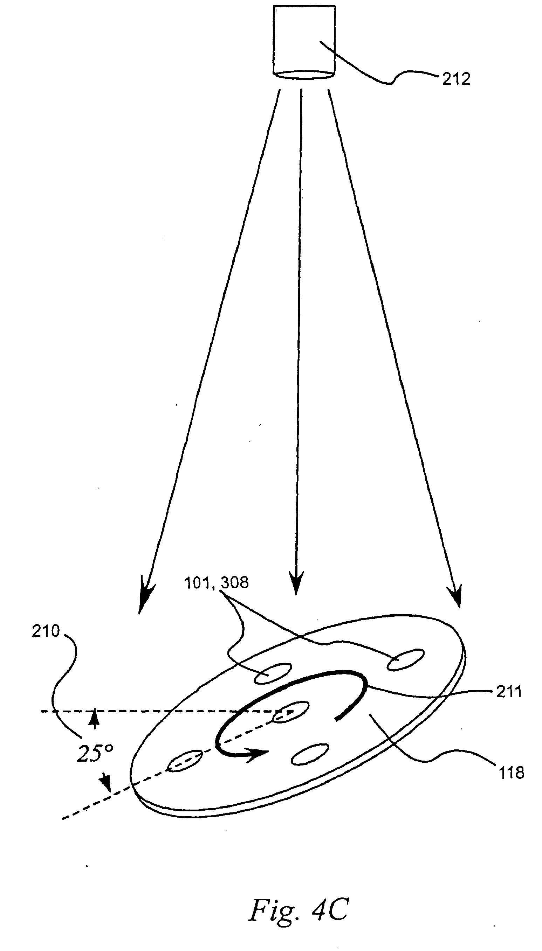

Fabrication of second perimeter electrode 105 is similar to the fabrication of first perimeter electrode 107. The second perimeter electrode element ranges in thickness from about 1 nm to about 10 nm, such as about 2 nm to about 9 nm, including about 3 nm to about 8 nm, about 4 nm to about 7 nm, and 5 nm to about 6 nm a 2 nm thickness of platinum. In some embodiments, the second perimeter element 105 will comprise platinum. The second perimeter electrode element 105 is formed by angled deposition as illustrated in FIG. 4C. Angle 210 is less than angle 207, for example 25 degrees, and source 212 is typically a vacuum evaporation source or a molecular beam epitaxy source or a sputtering source. The result of this deposition step is a layer in some embodiments 2 nm thick, and in some embodiments comprising platinum, which overhangs the edge of nanopore 101 as illustrated in FIGS. 1 and 2. Lithography and etching define the lateral extent of second perimeter electrode 105 as illustrated in FIG. 3F. Alternatively, a photolithography step may be performed prior to the deposition step as illustrated in FIG. 4C and the second perimeter electrode 105 may be defined by means of a lift-off process.

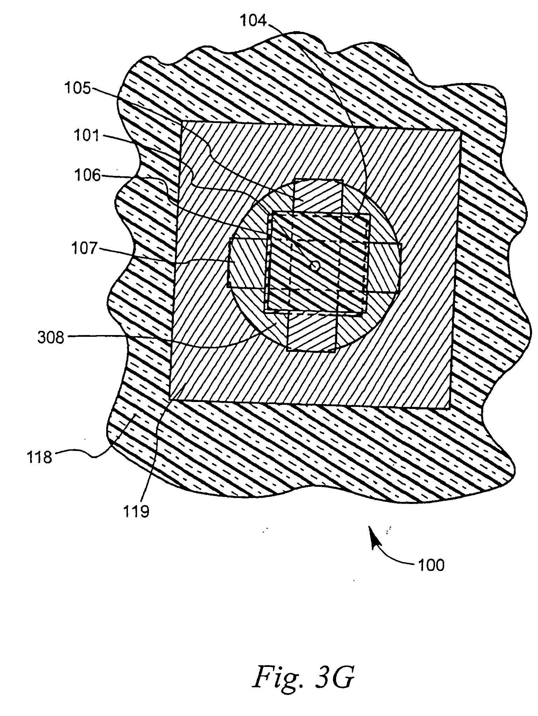

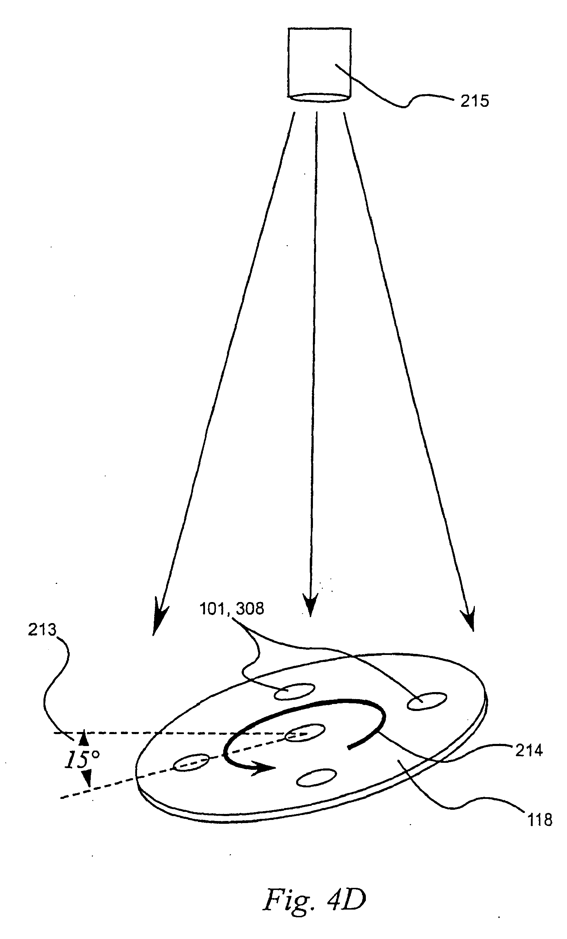

In certain embodiments, the device further includes a second insulator element 104. Fabrication of second insulator element 104 is similar to fabrication of first insulator element 106. An insulating layer is formed by angled deposition as illustrated in FIG. 4D. Angle 213 is less than angle 210, for example 15 degrees. Source 215 is typically a molecular beam epitaxy source or a sputtering source. The result of this deposition step is a layer in some embodiments 2 nm thick, and in some embodiments comprising silicon dioxide, which overhangs the edge of nanopore 101 but which, as illustrated in FIGS. 1 and 2, does not occlude overhanging perimeter of the second perimeter electrode 105 because the perimeter is shadowed from the deposition stream during the line-of-sight deposition. The lateral extent of optional second insulator element is defined by lithography and etching as illustrated in FIG. 3G. Alternatively, a photolithography step may be performed prior to the deposition step as illustrated in FIG. 4D and the second insulator element 104 may be defined by means of a lift-off process.

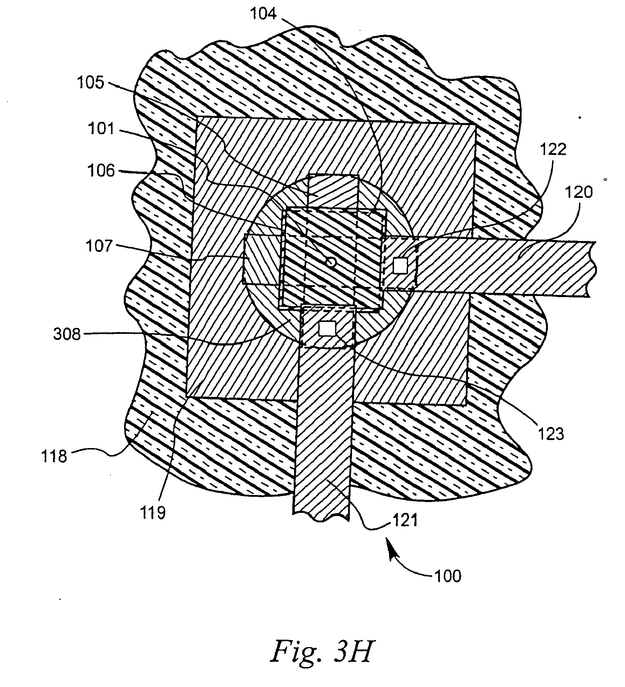

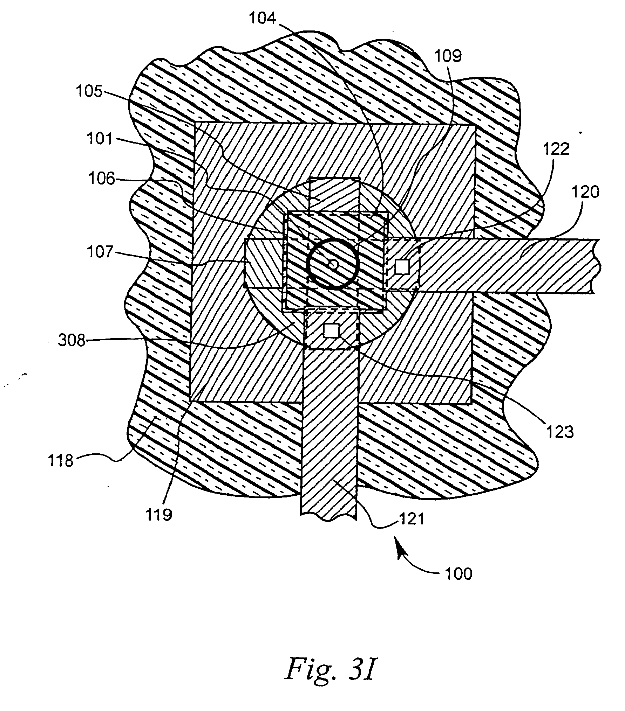

Next, as illustrated in FIG. 3H, electrical lead regions 120 and 121 are contacted to the first 107 and second 105 perimeter electrode regions at contact regions 123 and 122. Leads 120 and 121 are formed by standard IC techniques of metal deposition and lithography, for example by electron beam deposition of an aluminum layer followed by lithography and etching. Leads 120 and 121 may extend to contact pads, not shown, which provide for electrical contact to a circuit, not shown, similar to the circuit depicted for embodiment 100.

Next an optional insulator layer 109 is formed, for example by spinning on a layer of a polyimide precursor and curing that precursor to form layer of polyimide insulator, in order to provide electrical insulation over leads 120 and 121 and over the ends of the first 107 and second 105 perimeter electrodes.

The substrate 118 can then be diced by sawing to form individual nanopore chips, not shown, corresponding to substrate 108 as shown in FIG. 1 and FIG. 2. An individual nanopore chip can be connected to a fluidic apparatus to wet the first and second surfaces of the nanopore, and electrical connection of the chip to an electrical circuit can be performed.

The next step in the fabrication process is to seal the nanodimensioned passageway with a lipid bilayer 103. In placing the lipid bilayer onto the aperture, a bristle of sufficient dimensions, e.g. 10 to 200 µm diameter, usually 50 to 100 µm diameter, is dipped into a suitable lipid solution (e.g. lipid in organic solvent, concentration range from about 1 to 5 mg per ml, usually from about 2 to 4 mg per ml). The dipped bristle is then gently brushed against the nanodimensioned passageway, which results in the formation of a lipid bilayer 103 that seals the nanodimensioned passageway (see FIG. 3J). The seal is then tested and the aperture may be brushed repeatedly with a clean bristle until a desired bilayer 103 is obtained.

The final step in the preparation of the subject device is the insertion of the proteinaceous channel 102 into the lipid bilayer 103. Typically, an aqueous proteinaceous channel comprising solution is introduced into the first fluid containment member 110 and an electric field is applied across the lipid bilayer 103 in a manner sufficient for a proteinaceous channel 102 to insert or intercalate into the lipid bilayer 103.

It will be appreciated that the above fabrication process produces a series of edges of nanopore 101 defining a portion of the nanopore with successively smaller diameters. As noted in greater detail above, the inner walls of the nanopore 101 will define a frustum configuration. Each edge has an overhanging region, which can also be called a cornice, the bottom side of which is shadowed from a subsequent line-of-sight deposition so that it remains free of deposits, or nearly so. Thus, the nanodimensioned passageway when first formed in window 308 has a fourth edge with a fourth portion of the nanopore 101 extending there through. The first perimeter electrode 107 overhangs the fourth edge, forming a third edge with a third portion of the nanopore 101 extending there through. The third portion of the nanopore is smaller than the fourth portion of the nanopore. The first insulator element 106 overhangs the third edge, forming a first insulator edge with a second portion of the nanopore extending there through. The second portion of the nanopore is smaller than the third portion of the nanopore. The second peripheral electrode 105 overhangs the first insulator element edge, forming a first electrode edge with a first portion of the nanopore being smaller than the second portion of the nanopore. The optional second insulator element 104 overhangs the second peripheral electrode 105 edge, forming an insulator edge with a portion of the nanopore smaller than the first portion of the nanopore. Thus, beginning with a nanopore of initially large diameter and using the techniques of successive angled line-of-sight depositions, it is possible to end with a nanopore of small diameter. It will be appreciated that larger and smaller nanopores can be formed by varying the diameter of the edges, the thickness and number of deposited layers, and the angles of the successive depositions.

It will be appreciated that the particular details of the above structures and fabrication processes are representative only by way of example, and are in no way intended to be limiting. Many variations in structures and materials will occur to those skilled in the art without departing from the scope and spirit of the present invention. Additional layers may be added to the nanopore structure by the obvious extension of the techniques presented herein without departing from the scope and spirit of the present invention.

It will be appreciated that the electrode structures and fabrication techniques described herein have been presented with reference to the nanoscale, but that they may also possess utility at the larger microscale wherein the thicknesses of various layers are in the range of 100 nm to 25 µm.

It will be appreciated that the means of line-of-sight deposition chosen for any of the steps described abovemay result in some undesired deposition of insulator material onto electrode elements 107 or 105 or both, or may result in some undesired deposition of conductor material onto insulator regions 308, 106, or 104 or any combination of them. In such a case it may be possible to proceed with fabrication by using a known technique to remove the undesired deposition, especially if the undesired deposition is smaller in thickness than the desired layer thickness deposited on the first surface. Such known techniques for removal include, but are not limited to, chemical etching, ion beam milling, sputtering, plasma etching, and reactive ion etching.

### Uses of the Subject Devices

In general, the method of using the subject device 100 of the present invention includes applying an electrical voltage between the first 107 and second 105 perimeter electrodes of the device and monitoring the electrical current through the nanopore or monitoring the electrical current between first 107 and second 105 perimeter electrodes with an aim which may include detecting resonant tunneling current through a sample. In certain embodiments, the current flowing through the nanopore or sample is monitored and recorded over a period of time. Therefore, the monitoring provides a range of values representing the fluctuation of the current flowing through the nanopore.

In certain embodiments, when the device 100 is in use, first 110 and second 111 fluid containment members of the device are filled with a conductive ionic aqueous solution. In certain embodiments an electrical voltage may be applied between first 110 and second 111 fluid containment members. In certain embodiments, the monitoring of the electrical current through the nanopore or monitoring of the electrical current between first 107 and second 105 perimeter electrodes, or both, may be performed while a polymeric compound 112 is translocated through the proteinaceous channel 102 of the nanopore 101. In such embodiments, the fluid sample may be placed in the first 110 fluid containment member and polymeric compound present in the sample translocates the nanopore.

The polymeric compound 112 may comprise a variety of shapes, sizes and materials. The shape or size of the molecule is not important, but it must be capable of translocation through the proteinaceous channel 103 in the nanopore 101. For instance, both single stranded and double stranded RNA and DNA may be used as a polymeric compound 112. In addition, the polymeric compound 112 may contain groups or functional groups that are charged. Furthermore, metals or materials may be added, doped or intercalated within the polymeric compound 112 to provide a net dipole, a charge or allow for conductivity through the nanopre. The material of the polymeric compound 112 must allow for electron tunneling between electrodes.

Polymeric compound 112 is schematically depicted as a string of beads that is threaded through the proteinaceous channel 102 of the nanopore 101. The polymeric compound 112 typically resides in an ionic solvent such as aqueous potassium chloride, not shown, which also extends through nanopore 101. It should be appreciated that, due to Brownian motion if nothing else, polymeric compound 112 is always in motion, and such motion will result in a time-varying position of each bead 113 within proteinaceous channel 102 of the nanopore 101. The motion of polymeric compound 112 will typically be biased in one direction or another through the pore by providing an external driving force, for example by establishing an electric field through the pore between a set of electrodes, not shown.

### Utility

The subject devices find use in a variety of different applications in which the ionic current through a nanopore is monitored or resonant tunneling current through a sample is monitored. Representative applications in which the subject devices find use include, for example, separation of molecules, capturing of molecules, characterization of polymeric compounds, e.g. the determining of the base sequence of a nucleic acid; and the like.

In some embodiments, the subject devices 100 of the present invention are useful in characterizing a polymeric compound 112, such as DNA. Once the fluid sample containing the polymeric compound 112 is in close proximity to the nanopore, for example, is present in region 110 of the device 100, the polymeric compound present in the fluid sample moves relative to the nanopore in a manner such that each different monomeric unit 113 of the polymeric compound 112 causes a correspondingly different time-varying current to flow between first 107 and second 105 perimeter electrodes. in response to the application of a time-varying voltage applied between first 107 and second 105 perimeter electrodes.

For example, a single stranded nucleic acid may be translocated through the proteinaceous channel positioned in the nanopore and the effect of each base on the current flowing between first 107 and second 105 perimeter electrodes monitored and recorded, thereby providing a range of values representing the fluctuation of the current flowing between first 107 and second 105 perimeter electrodes as each monomeric unit 113 of the polymeric compound 112 translocates through the nanopore. Such a range of fluctuations over a period of time may then be analyzed to determine the identity of the monomeric units of the polymeric compound.

As described above, the voltage generating element may be ramped, i.e., by application of a time-varying voltage, in order to provide a resonant tunneling current through a sample. The general principle is to ramp the tunneling voltage across the electrodes over the energy spectrum of the translocating polymeric compound. At specific voltages the incident energy will sequentially match the internal nucleotide energy levels, giving rise to a detectable change, e.g., increase, in the observed tunneling current. In representative embodiments, the ramp-time of the applied voltage is short compared to the nucleotide translocation time through the nanopore. For example, in certain embodiments, the applied tunneling voltage frequency may be in excess of about 10 MHz.

As a monomer translocates through the nanopore and between the two perimeter electrodes, it will always pass a point where the barriers separating it from the two perimeter electrodes are equal, regardless of the origin of the initial barrier asymmetry (either spatial separation or steric asymmetry). At this point, there will be a detectable resonant tunneling current increase as the tunneling voltage scans the internal energy spectrum of the individual monomer. As previously discussed, each type of monomer unit 113 has a characteristic internal energy level spectrum which would allow it to be distinguished from the other monomer unit 113 types.

The applied voltage and tunneling current can be seen to produce a defined signal that is indicative of the portion of the biopolymer that is in a matched-barrier position between first 107 and second 105 perimeter electrodes. Each monomeric unit 113 of the polymeric compound 112 will produce a differing signal in the tunneling current over time as the varying voltage is applied. For instance, when each monomeric unit 113 or portion of the polymeric compound 112 is positioned such that the barriers are symmetric, a larger overall signal can be seen from the tunneling current as opposed to when the barriers are asymmetric. These differing signals provide a spectrum of the portion of the polymeric compound 112 that is positioned in a matched-barrier position between first 107 and second 105 perimeter electrodes. These spectra can then be compared by computer to previous spectra or "finger prints" of nucleotides or portions of the polymeric compound 112 that have already been recorded, i.e., a reference or control. The residue of the polymeric compound 112 can then be determined by comparison to this reference or control, e.g., that may be in the form of a database. This data and information can then be stored and supplied as output data of a final sequence.

From the resultant recorded current fluctuations, the base sequence of the nucleic acid can be determined. Methods of characterizing polymeric molecules in this manner are further described in application Ser. No. 08/405,735 and entitled Characterization of Individual Polymer Molecules Based on Monomer-interface Interactions, the disclosure of which is herein incorporated by reference.

Results obtained from such methods may be raw results, such as signal lines for the signal producing system of the device. In the alternative, the results may be processed results, such as those obtained by subtracting a background measurement, or an indication of the identity of a particular residue of a polymeric compound (for example an indication of a particular nucleotide or amino acid.

It will be appreciated that the utility of the structures and processes described herein has been discussed with respect to the theory of resonant tunneling, but that the utility of these structures and processes is in no way limited to resonant tunneling, but instead applies also to other physical phenomena useful for measurement and manipulation of small object including biopolymers, including but not limited to non-resonant tunneling, electrostatic attraction and repulsion, fluidic field effect transistors, electrolysis, and the like. Either one or both of the perimeter electrodes 105 and 107, or the insulator element 106 between perimeter electrodes 105 and 107, might be coated with a monolayer of a molecule useful for binding to or detecting a biopolymer molecule of interest.

Representative applications for use of nanopore devices are further described in US Patent Nos. 5,795,782, 6,015,714, 6,362,002, 6,464,842, 6,627,067, 6,673, 615, 6,674,594, 6,706,203, 6,706,204, 6,783,643, 6,267,872, US Patent Publication Nos. 2003/0044816, 2003/0066749, 2003/0104428, WO 00/34527; the disclosures of which are herein incorporated by reference.

In certain embodiments, the subject methods also include a step of transmitting data or results from the monitoring step, as described above, to a remote location. By "remote location" is meant a location other than the location at which the translocation occurs. For example, a remote location could be another location (e.g. office, lab, etc.) in the same city, another location in a different city, another location in a different state, another location in a different country, etc. As such, when one item is indicated as being "remote" from another, what is meant is that the two items are at least in different buildings, and may be at least one mile, ten miles, or at least one hundred miles apart.

The preceding merely illustrates the principles of the invention. It will be appreciated that those skilled in the art will be able to devise various arrangements which, although not explicitly described or shown herein, embody the principles of the invention and are included within its spirit and scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope and spirit of present invention is embodied by the appended claims.

## Claims

1. A nanopore device comprising:
a first fluid containment member;
a second fluid containment member;
a fluid barrier separating said first and second fluid containment members;
a nanopore present in said fluid barrier and comprising first and second perimeter electrodes separated by an insulator element; and
a biopolymeric channel positioned in said nanopore.

2. The device according to claim 1, wherein said nanopore has inner walls configured to define a frustum.

3. The device according to claims 1 or 2, wherein said nanopore has a top inner diameter that is smaller than a bottom inner diameter.

4. The device according to claims 1, 2 or 3, wherein said first and second perimeter electrodes are part of a resonant tunneling sensor.

5. The device according to claims 1, 2, 3 or 4, wherein said device further comprises an element for applying an electrical voltage between said first and second perimeter electrodes.

6. The device according to any of the preceding claims, wherein said device further comprises an element for measuring an electrical current between said first and second perimeter electrodes.

7. The device according to any of the preceding claims, wherein said biopolymeric channel is a proteinaceous channel.

8. The device according to claim 7, wherein said proteinaceous channel comprises α-hemolysin.

9. The device according to any of the preceding claims, wherein said channel is held in position with a lipid bilayer.

10. A method for fabricating a nanopore in a solid substrate, comprising:
(a) producing a nanodimensioned passageway through a planar solid substrate;
(b) positioning an electrode element about an opening of said passageway, wherein said electrode element comprises first and second perimeter electrodes separated by an insulator element; and
(c) positioning a channel in said nanodimensioned passageway;
to produce said nanopore.

11. A method comprising:
applying an electrical voltage between first and second perimeter electrodes of a device according to any of claims 1 to 9, and
monitoring an electrical current between said first and said second perimeter electrodes.
